(19) **Europäisches Patentamt / European Patent Office / Office européen des brevets**

(11) **EP 1 360 904 B1**

(12) **EUROPEAN PATENT SPECIFICATION**

(45) Date of publication and mention
of the grant of the patent:
**16.01.2008 Bulletin 2008/03**

(51) Int Cl.:
*A23K 1/16* (2006.01)  *A23K 1/18* (2006.01)
*A61K 9/50* (2006.01)

(21) Application number: **03007601.2**

(22) Date of filing: **02.04.2003**

(54) **Protected compound formulations of amino acids and process for their preparation**

Formulierungen von geschützten, aus Aminosäuren bestehenden aktiven Substanzen und Verfahren zu ihrer Herstellung

Formulations de composés actifs protégés constitués d'acides aminés et leur procédé de préparation

(84) Designated Contracting States:
**AT BE BG CH CY CZ DE DK EE ES FI FR GB GR HU IE IT LI LU MC NL PT RO SE SI SK TR**

(30) Priority: **10.05.2002 DE 10220785**

(43) Date of publication of application:
**12.11.2003 Bulletin 2003/46**

(73) Proprietor: **Evonik Degussa GmbH**
**40474 Düsseldorf (DE)**

(72) Inventors:
• **Hasseberg, Hans-Albrecht, Dr.**
**63584 Gründau (DE)**
• **Heimbeck, Winfried, Dr.**
**63776 Mömbris (DE)**
• **Greissinger, Dieter, Dr.**
**61194 Niddatal (DE)**
• **Möse, Wolfgang**
**63505 Langenselbold (DE)**
• **Polzer, Wolfgang**
**63584 Gründau (DE)**
• **Shi, Nongyuan, Dr.**
**63457 Hanau (DE)**
• **Körfer, Martin, Dr.**
**2920 Kalmthout-Achterbroek (BE)**

(56) References cited:
EP-A- 0 406 041       EP-A- 0 427 639
EP-A- 0 447 297       EP-A- 0 495 349
EP-A- 0 588 346       US-A- 4 876 097
US-A- 4 877 621       US-A- 5 290 560

**Description**

The invention relates to protected active compound formulations of amino acids, in particular methionine, and of vitamins, pigments or enzymes and a process for the preparation of these protected active compound formulations. This invention relates in particular to active compound formulations of amino acids which are suitable for providing amino acids in a rumen-protected form for the nutrition of stock animals, in particular ruminants.

Methionine or its substitute, the methionine hydroxy analogue, have been employed very successfully for decades in animal nutrition as additives for meeting the requirement of stock animals for the essential amino acid in a economical but also in a physiologically and ecologically optimum manner. However, methionine or also the hydroxy analogues cannot be employed directly in the nutrition of ruminants, since the microbes living in the rumen metabolize amino acids, hydroxy acids and proteins relatively rapidly. If unprotected amino acids or hydroxy acids are fed to ruminants, the predominant proportion of these amino acids or hydroxy acids would therefore not be available to the ruminant for its own supply, but would be lost for protein synthesis in metabolism by microbial breakdown in the rumen.

A large number of so-called rumen-protected products are known in the prior art, but a predominant number thereof are not stable enough to microbial breakdown in the rumen to ensure an appropriate use as an additive. The best products to date are based on the use of relatively large shaped pieces of methionine, such as, for example, granules or pellets, which are protected by coating with polymers. Ethylcellulose (EP 0 495 349) or a copolymer of vinylpyridine and styrene (US Patent 4,877,621 and US Patent 4,876,097) is used, for example, as the coating composition.

The products described in EP 0 495 349 comprise a shaped piece of methionine which is obtained by pelleting methionine and auxiliary substances and is then coated with a protective shell of ethylcellulose, preferably in 2-layer application with an inorganic auxiliary substance or filler (e.g. sodium aluminum silicate). The cylindrical shape of the pellets resulting from the preparation leads to edges with a relatively thin coating, which are regarded as intentional breaking points and as essential for the release of the active compound.

EP 0 495 349 (comparison example 5) furthermore reports that rounded-off particles with a uniformly thick film thickness show adverse effects. -

The products obtained in EP 0 495 349 release methionine in the entire digestive tract, rel. little in the rumen, which can be seen from the high in vitro rumen protection of ≥ 80%, but increasingly more in the further digestive tract of the abomasum and small intestine. These delayed release properties, also called "slow release" properties, are preferred here since a small introduction of methionine in the rumen stabilizes the rumen flora. This is favorable overall, and a slow uniform release of methionine in the further course of digestion leads to a more uniform utilization than a sudden pH-controlled release in the abomasum at pH 2, which is aimed for with the products from US Patent 4,877,621 and US Patent 4,876,097.

On the basis of the size of the particles from EP 0 495 349 of approx. 3.5 mm length, however, stable homogeneous distribution of such products in feed mixtures is not always easy to achieve, on the one hand because of the small number of particles, due to the size, per volume and on the other hand because of the difference in size with respect to the other mixed feed components, which can lead to demixing in some cases under adverse conditions. On the other hand, there is no mechanical stability when exposed to very high stresses, such as e.g. during pelleting of the feed.

The protected products described in US Patent 4,877,621 and US Patent 4,876,097 are based on coating of pellets which comprise amino acids and have diameters of 0.1 to approx. 5 mm, a pH-sensitive protective shell being built up. Since the intention is to produce pH-dependent release properties, a copolymer of 2-vinylpyridine and styrene is always used here as an essential constituent, and in US Patent 4,876,097 additionally further components, such as e.g. zein cellulose acetobutyrate, polyvinyl acetate, chitosan, ethylcellulose and further auxiliary substances, including various solvents, which leads to a complicated protective shell comprising several components. The release properties indeed show entirely good results, but are achieved at the expense of a high complexity and by the use of coating or auxiliary substances which in some cases have not yet been approved in feedstuffs legislation, which makes economical utilization difficult.

EP 427639, EP 406041 and EP 447297 describe coated granules of methionine or methionine/lysine which also have pH-controlled release properties.

EP 0 614 615 relates to a feedstuffs additive of a rumen-protected composition which comprises a biologically active substance and a protective shell and furthermore also has a pelleting-resistant protective shell of natural or synthetic polymers. Polymers which are suitable for this pelleting-resistant protective shell have an elasticity modulus at 30°C of between $10^8$ and $10^{11}$ dynes/cm$^2$ and a glass transition temperature of between 50°C and 150°C. This composition comprises pelleted granules with a particle size of greater than 1.5 mm.

US Patent 5,290,560 describes a process for the preparation of granules by extrusion with food or medicament active compounds for ruminants, in which the core of the granules is extruded in a first step and the core is coated by pulverization in a second step.

EP 588 346 describes a process for the preparation of 1.5 mm granules by extrusion with food or medicament active compounds for ruminants, in which the core of the granules is extruded in a first step and the core is coated by fluidized bed coating in a second step. The feedstuffs additive composition for ruminants described here comprises a coated core of the biologically active substance. The biologically active substance is an amino acid, such as e.g. lysine or methionine. The coating material comprises hydrogenated animal fat, oil or wax and an aliphatic monocarboxylic acid and/or nucleic acid, nucleotides, nucleosides, bases containing nucleic acid or salts.

In view of the problems described, it was an object to provide an active compound formulation which does not have the above-mentioned disadvantages of the existing products or has them to a significantly smaller extent and shows the advantages of the "slow release" properties described in EP 495349, that is to say an increased release after > 6 h ($\cong$ residence time in the rumen).

One possibility of achieving the object described chiefly comprises coating of significantly smaller particles. However, this possibility on the one hand has the disadvantage of increased consumption of often relatively expensive coating compositions. On the other hand, multilayer application with additional components (fillers), which allow later dissolution of the protective shell at the desired place, such as is necessary in the case of the products described in EP 0 495 349, can scarcely be realized with very small particles (see EP 0 614 615, comparison examples 1-6).

The object of this invention is therefore to provide a protected active compound formulation which is based on amino acids, in particular methionine or salts thereof, and has a particle diameter of less than 1000 $\mu$m. Furthermore, a rumen-protected active compound formulation should be coated with a protective shell and by one-layer application, so that analogous rumen protection rates compared with the products e.g. from EP 0 495 349 and distribution properties in the mixed feed which are moreover improved are obtained.

This rumen-protected active compound formulation based on amino acids should meet the requirement of being able to be absorbed to an increased extent under the conditions of the subsequent abomasum or at the latest in the small intestine of the ruminant.

Active compounds which are employed are, for example, amino acids or salts thereof, vitamins, pigments or enzymes.

In addition, the formulation should have the highest possible mechanical resistance, so that no relatively large losses of intactly protected particles occur during the typical processes of preparation of mixed feeds, such as mixing, conveying or pelleting.

This requirement profile should furthermore as far as possible be achieved only with the aid of auxiliary substances which are approved in feedstuffs legislation and in the end move the active compound formulation into a price frame which does not exceed that of the current products on the market. These requirements are summarized in the following:

- In vitro release after 6 hours:

    corresponds to the protection rate in the rumen, i.e. a maximum of 25% release/digestion of the biologically active substance in the rumen at a pH of 5.5-6.5, in approx. 6 hours (h) and in the presence of microbes, protozoa;

- In vitro release after 6-24 hours:

    corresponds to release in the further digestive tract after a residence time of more than 6 h to 24 h and should be 35 - 65% of the biologically active substance initially contained in the product,

- The active compounds and coating materials should withstand the conditions of feed processing of mixing, conveying and pelleting (approx. 80°C - 100°C, steam, pressure, shear forces).

The product according to the invention meets these conditions.

It has furthermore been found that the "slow-release" active compound formulations according to the invention are also resistant to the oxidation action of air, the effects of water or of trace elements, e.g. during storage in bulk or in a feed mixture. The active compound formulations according to the invention can moreover be employed in other uses in which a controlled "slow-release" type of release is desirable, such as e.g. in the case of vitamins, pigments or enzymes.

The object of this invention is furthermore to provide a process for the preparation of these protected active compound formulations.

This object is achieved in respect of the active compound formulation in that the desired active compound, in particular methionine, is employed directly in the form of particles or crystals with a diameter of less than 1 mm (1000 $\mu$m) without a prior granulation step, or spray granules with a diameter of less than 1 mm. The particle diameter is that before the coating. The shape of the particles to be processed is not particularly critical, it is therefore possible to employ both commercially available platelet-shaped crystals and circular crystals. It has been found that e.g. methionine particle coated according to the invention with a diameter of less than 1 mm have analogous rumen protection rates compared with the products described in EP 0 495 349 (comparison example 5).

The invention is best described by claim 1 and 17.

The active compound formulations according to the invention in general comprise small coated active compound particles in the particle size range of 100 to 1000 μm, in particular 200 to less than 800 μm, preferably 300 to 700 μm, particularly preferably 400 to 600 μm.

The active compound formulation according to the invention are coated with at least one protective shell. The protective shell consists of a single coating material Active compound formulations with a single protective shell produced by one-layer application are preferred.

Coatings based on film-forming agents comprising naturally occurring or modified naturally occurring polymers or homo- and copolymers which can be prepared by conventional known processes are suitable for the protective shell. Such suitable polymers are, for example, cellulose esters, ethers, acrylates, poly(meth)acrylates, polyamides, polyesters or copolymers of e.g. acrylonitrile, styrene, ethylene, propylene, butadiene, or esters and amides of methacrylic acid or acrylic acid.

A coating based on cellulose ethers, preferably ethylcellulose, is very particularly suitable. It is particularly preferable to use ethylcellulose dissolved in ethanol, since a coating with a content of 4 to 30 per cent by weight (wt.%), preferably 5 - 20 wt.% and particularly preferably 9 - 15 wt.% ethylcellulose, based on the end product, can be achieved in this manner.

Various ethylcellulose types can be employed as the coating composition according to the invention. Thus, it has been possible to employ ethylcellulose types of varying viscosity according to the different degree of substitution all from Hercules (N10, N50, T50, X200) by way of example, and to achieve similarly advantageous results here.

The ethylcellulose types investigated here by way of example correspond to various viscosity classes. The number corresponds to the dynamic viscosity in mPa*s measured in a 5% solution in toluene/ethanol 80:20 (w/w) at 25ºC.

The active compound formulations according to the invention can be produced in an advantageous manner by fluidized bed coating, but optionally also by other processes of microencapsulation, such as e.g. coacervation.

It has been possible here, in an unforeseeable manner, to dispense with the use of further inorganic or organic auxiliary substances, such as e.g. sodium aluminum silicate or the Na salt of carboxymethylcellulose or other substances described in the above-mentioned patents, which leads to a significant simplification of the process and of the active compound formulations and to the direct utilizability of such active compound formulations without prior, tedious feedstuffs legislation approval processes.

It has been found that methionine crystals in particular can be employed directly without a prior granulation step. It is possible here to employ both platelet-shaped crystals and round crystals of conventional commercial quality.

The preferred particle size of the active compound formulation of 400 - 600 μm for the subsequent coating can be achieved by simple operations such as sieving, air sifting or another separation process from the prior art, it as a rule being sufficient to carry out a single separation operation, such as e.g. a sieving over a 400 μm sieve, because the commercially available methionine products scarcely have contents above 600 μm.

To produce round crystals, if this is desired, methionine particles with the conventional platelet shape can optionally also be rounded off or granulated, with or without the presence of further auxiliary substances. The consumption of coating composition is lowest in the case of round particles.

It is also possible to employ spray granules prepared from methionine solutions or methionine salt solutions, such as e.g. Na, K, Ca, Mg , Zn methionate in pure or mixed form, which have a rounded-off shape due to the preparation.

To prepare the active compound formulations according to the invention by fluidized bed coating, e.g. DL-methionine, for example, in the particle size range of significantly smaller than 1000 μm is initially introduced into a fluidized bed apparatus and sprayed with a coating composition - preferably ethylcellulose - dissolved in an organic solvent. The conditions are adjusted such that a covering of the particles which is as complete as possible is achieved with minimal agglomeration.

The preparation of the active compound formulations according to the invention and the test methods used for their characterization are described in the following.

Examples 1-9 and comparison examples A and B

Determination of the release properties

$$\text{Rumen protection rate [\%] =} \quad \text{Content of the still protected methionine after an incubation time of 6 to 24 h in a test medium (water pH 5.5 or buffer solution).}$$

Release test (in vitro) for active compound formulations with pH-independent release properties

The active compound formulation is shaken in desalinated water at 37°C/pH 5.5 in a vibrating water-bath - desired value typical for product A (protected methionine, pellet-shaped approx. 1.8 * 3.5 mm, analogously to ex. 11 from EP 0 495 349)

- for 6 h ($\cong$ rumen)                    high $\geq$ 80%

- for 24 h ($\cong$ rumen and further digestive tract) low                    approx. $\leq$ 45%

After the particular period of time in the vibrating water-bath, the solutions were filtered, the methionine released from the protected active compound formulations was determined quantitatively in the filtrate (by bromide/bromate titration) and the protection rate was calculated according to the following formula:

$$\text{Protection rate [\%] = 1- \% methionine released/\% methionine starting amount)*100}$$

The use, preferred here, of desalinated water instead of the buffer mixtures described in EP 0 495 349 leads to equivalent results in investigations on pH-independent "slow release" systems (comparison example B).

The in vitro protection rates after incubation for 6 h measured in this manner have a tendency to correlate well according to EP 0 495 349 with results of in vivo experiments. On the other hand, the in vitro protection rates after incubation for 24 h are not identical to the protection rates or corresponding release rates to be determined in living systems. As EP 0 495 349 shows, in vitro protection rates of 30-40% after 24 h (corresponds to release of approx. 45-55%) correspond to good in vivo bioavailabilities (= methionine content in blood plasma), so that the 24 h laboratory value is to be seen here as a guideline parameter.

Determination of the stabilities towards pelleting

Preparation of the mixtures

Milk yield feed ML 183 (Raiffeisen, Wiesbaden) was mixed together with the corresponding amounts of protected methionine active compound formulation according to the invention in a 50 L Lödige mixer for 4 min at 100 rpm. The finished mixtures were employed for pelleting.

The preparation of feed pellets was carried out in a Walther EI 15 press under the pelleting conditions stated in table I. This was first started up with feed containing no active compound. After a constant press run had been reached, the mixtures were pressed successively with various test products.

The pellets were cooled slowly, the fine content (particle size < 3 mm) was sieved off and the finished batches were unloaded.

**TABLE I**

| PELLETING CONDITIONS | |
|---|---|
| Press | Walther EI 15 |

(continued)

| PELLETING CONDITIONS | |
| --- | --- |
| Die bore | 5.5 mm |
| Channel length | 30 mm |
| Current uptake | 6.3 - 6.8 A |
| Pelleting temperature | 65-75°C |
| Steam pressure | 8 bar |
| Concentration of test substance | 3% DL-methionine weight equivalent in milk yield feed |
| Pellet size | 5.5 * 10 - 12 mm |
| Residual moisture content after cooling | approx. 11% |

The protection rates were measured on the feed pellets prepared in this way in a vibrating water-bath as described above. The methionine released was quantified here by HPLC chromatography.

Preparation of the protected methionine particles

The experiments were carried out in a laboratory fluidized bed granulating unit. In each case 1 kg of sieved methionine (particle range as stated in table II) per experiment was initially introduced into the fluidized bed apparatus as the starting material. Either platelet-shaped crystals (Degussa AG) or crystals with a round particle shape (Sumitomo) were used. Various ethylcellulose types from Hercules Aqualon (viscosity class N10, N50, T50, X200 as stated in table II) were employed for the coating. Ethylcellulose was sprayed as a 5% ethanolic solution through a nozzle from the top on to the methionine particles held underneath in the fluidized bed.

Comparison examples A and B were coated in accordance with EP 0 495 349 with 2 layers of different coating materials.

Nitrogen was employed as the drying and atomizing gas and the unit was kept in the non-explosive range in this manner. The following test settings were used:

| | |
| --- | --- |
| Intake temperature: | 110°C |
| Bed temperature: | 85 - 90°C |
| Waste air temperature: | 77 - 80°C |
| Inflow volume flow: | 90 mN$^3$/h |
| Atomization pressure: | 3 bar |

The consumption of coating composition was monitored continuously and intermediate samples were taken from the fluidized bed apparatus at regular intervals of time. Both on the intermediate samples and on the end sample, the methionine content was determined and a release test was carried out to determine the protection rates.

In this manner it was possible to determine the protection rates as a function of the methionine content and of the complementary content of coating material for each combination of methionine and coating material. All the results in this respect are listed in table II.

By pelleting the active compound formulations according to the invention in milk yield feed and subsequent repetition of the release test, the protection rate after pelleting was additionally determined as a measure of the stability towards pelleting (table II).

**Table II**

| Example / comparison example no. | Methionine quality | Coating material Content, %, type | Methionine content % | Protection rates (in vitro) | | | |
|---|---|---|---|---|---|---|---|
| Methionine active compound formulations protected by coating | | | | | | | |
| | | | | Rumen after 6 h, % | Total after 24 h, % | Release 6 h: 24h, % | After pelleting 6 h, % |
| 1 | platelets | 8.3, EC N10 | 91.7 | 22 | 5 | 17 | nm[1] |
| | 400-600 μm | 16.6, EC N10 | 83.4 | 73 | 40 | 33 | |
| 2 | platelets | 4.7, EC N50 | 95.3 | 6 | 3 | 3 | nm[1] |
| | 400-600 μm | 12.0, EC N50 | 88.0 | 63 | 22 | 41 | |
| 3 | platelets | 19.9, EC N50 | 80.1 | 80 | 53 | 27 | 30 |
| | 400-600 μm | | | | | | |
| 4 | round crystals | 4.3, EC N50 | 95.7 | 49 | 18 | 31 | |
| | 400-600 μm | 6.8, EC N50 | 93.2 | 69 | 39 | 30 | |
| | | 8.7, EC N50 | 91.3 | 79 | 52 | 27 | |
| | | 11.2, EC N50 | 88.8 | 83 | 60 | 23 | |
| | | 13.2, EC N50 | 86.8 | 89 | 71 | 18 | 26 |
| A | platelets 400-600 μm | 5.6, EC N50 2.2, Wessalith P | 90.7 | 26 | 6 | 20 | nm[1] |
| 5 | platelets | 7.5, EC T50 | 92.5 | 35 | nm[1] | nm[1] | |
| | 400-600 μm | 10.4, EC T50 | 89.6 | 54 | 15 | 39 | |
| | | 14.2, EC T50 | 85.8 | 71 | 32 | 39 | |
| | | 17.6, EC T50 | 82.4 | 81 | 46 | 35 | |
| | | 20.9, EC T50 | 79.1 | 86 | 56 | 30 | 34 |
| 6 | platelets | 6.6, EC X200 | 93.4 | 26 | nm[1] | nm[1] | |
| | 400-600 μm | 8.9, EC X200 | 91.1 | 42 | 10 | 32 | |
| | | 14.4, EC X200 | 85.6 | 66 | 32 | 34 | |
| | | 17.8, EC X200 | 82.2 | 75 | 49 | 26 | |

(continued)

| Example / comparison example no. | Methionine quality | Coating material | Methionine content % | Protection rates (in vitro) | | | |
|---|---|---|---|---|---|---|---|
| | | Content, %, type | | Rumen after 6 h, % | Total after 24 h, % | Release 6 h: 24h, % | After pelleting 6 h, % |
| | | 18.5, EC X200 | 81.5 | 77 | 46 | 31 | 34 |
| 7 | round crystals 100-300 μm | 6.0, EC N50 | 94.0 | 13 | nm[1] | nm[1] | |
| | | 12.9, EC N50 | 87.1 | 57 | 17 | 40 | |
| | | 15.6, EC N50 | 84.4 | 66 | 27 | 39 | |
| | | 15.8, EC N50 | 84.2 | 63 | 30 | 33 | 8 |
| 8 | round crystals 100-300 μm | 4.9, EC T50 | 95.1 | 14 | nm[1] | nm[1] | |
| | | 10.2, EC T50 | 89.8 | 55 | 21 | 34 | |
| | | 15.1, EC T50 | 84.9 | 74 | 42 | 32 | 8 |
| 9 | platelets 100-300 μm | 7.9, EC N50 | 92.1 | 27 | nm[1] | nm[1] | |
| | | 14.5, EC N50 | 86.5 | 23 | nm[1] | nm[1] | |
| | | 16.9, EC N50 | 84.1 | 37 | nm[1] | nm[1] | |
| | | 19.2, EC N50 | 80.8 | 41 | 7 | 34 | |
| | | 21.6, EC N50 | 78.4 | 37 | 9 | 28 | nm[1] |
| B | Pellets analogously to ex. 11 from EP 0495349 1800-3500 μm | 4.6, EC N50 2.3, Na Al silicate | 84.8 | 82 | 42 | 40 | 15 |
| [1]nm = not measured | | | | | | | |

As can easily be seen from table II, when sieved platelet-shaped methionine with a particle size of 400 - 600 μm is employed at an ethylcellulose application of approx. 17%, protection rates of 81% after 6 h and 46% after 24 h are found in the standard test (see ex. 5). With round DL-methionine crystals, rumen protection rates of 79% after 6 h and 52% after 24 h were already found with an ethylcellulose application of 8.7% (see ex. 4).

In both cases, the protection rates were in the range of the values mentioned in EP 0 495 349. In the case of the rounded-off crystals, approximately the same protection rates as in the case of the platelet-shaped crystals were achieved with about half the application amount.

After pelleting in milk yield feed, it was still possible to obtain in vitro rumen protection rates of up to 34% after 6 h (ex. 5, 6), which means a significant improvement compared with the products from EP 0 495 349, which are less stable to pelleting because of their size (protection rate after pelleting (6 h): 15%, comparison example B). Protected active compound formulations with a particle size of 100 -300 μm of the methionine particles employed led to protection rates after pelleting which were again lower, which suggests that the optimum range has already been left again here. (Example 7 and 8, protection rate after pelleting (6 h): 8%)

Protection rates after 6 h of at least 60%, preferably of more than 70%, in particular of more than 80%, and by comparison with this correspondingly lower protection rates after 24 h, which are at least 30%, preferably at least 35%, but in particular more than 40% below those after 6 h, have proved, according to the disclosure of EP 0 495 349, to be a prerequisite for the bioavailability (increase in the methionine blood plasma level) to ruminants which is described there.

In principle, a higher protection rate is achieved with a higher layer application. However, too high a protection has the effect in turn that too little of the protected amino acid is available to the animal. On the basis of the disclosure in EP 0 495 349, it was to be assumed that round particles do not bring about favorable release properties because of their uniform layer thickness. However, against expectation it has been found that small round and also platelet-shaped particles with a particle size of between 100 μm and 1000 μm which are coated with a single covering with a single protective shell, in particular of ethylcellulose have favorable release properties equivalent to the products from EP 0 495 349, as shown, in particular, by the comparison of examples 3, 4, 5, 6 with comparison example B.

In addition to the release properties and the mechanical stability, it is also important that feed mixtures show as little tendency towards demixing as possible during handling, that is to say the lowest possible demixing on trickling. To obtain information on the demixing properties of the active compound formulations according to the invention during trickling compared with conventional products, these were mixed into the usual feed for ruminants and a trickling demixing test was carried out. In this, the initially homogeneously mixed feed mixtures were allowed to trickle through a discharge funnel and a poured cone was produced in this manner under standardized conditions, samples being taken in this at various defined points for analysis of the methionine content. This trickling demixing test is described in examples 10-25 and comparison examples C and D.

Examples 10 to 17, comparison examples C-F

Determination of the tendencies towards demixing

Mixtures of the active compound formulations according to the invention with the usual feedstuffs were prepared. For this, both commercially available mineral feed, such as Blattin (Höveler) and Spur-a-min (Höveler) and organic feed such as soya meal (Raiffeisen) and milk yield feed (Raiffeisen) were mixed with protected methionine active compound formulations according to the invention as stated in table III and a uniform mixture was produced in a tumble mixer. The particle size distributions of the feed types employed is stated in table IV.

The mixtures produced in this manner were investigated for their tendencies towards demixing during trickling in the following test. For this, the mixtures were in each case transferred to a glass funnel and allowed to run out from a falling height of 10 cm on to a flat base. The dimensions of the funnel were: diameter 100 mm, cone height 90 mm, diameter of the discharge 7 mm, discharge length 90 mm, falling height from the discharge 100 mm.

The cone formed in each case was divided into 3 layers of equal height, the layer produced in each case was ground (< 250 μm) and the methionine content was determined in each layer by HPLC chromatography. From the 3 methionine contents of the 3 layers, the variation coefficients was determined as a measure of the demixing during trickling. The higher the particular variation coefficients found, the greater the particular demixing during trickling. The smaller the variation coefficients, the lower the demixing of the particular product from the particular feed mixture during trickling. The results are summarized in table V.

The variation coefficient (VC) of the trickling demixing experiments were calculated according to the following formula:

$$\text{VC [\%]} = (\text{standard deviation of the individual measurements/mean of the individual measurements}) *100$$

**Table III**

**ACTIVE COMPOUND FORMULATIONS WITH THE USUAL FEEDSTUFFS**

| Example/com-parison example no. | 10 | 11 | C | 12 | 13 | D |
|---|---|---|---|---|---|---|
| Batch with | | | | | | |
| Blattin, g | 200 | 200 | 200 | | | |
| Spur-a-min, g | | | | 200 | 200 | 200 |
| End product from ex. 5 (79.1% methionine), g | 25.28 | | | 25.28 | | |
| End product from ex. 4 (86,8% methionine), g | | 23.04 | | | 23.04 | |
| Product A[1] (86.6% methionine) | | | 22.91 | | | 22.91 |
| Methionine content (calculated), % | 8.9 | 8.9 | 8.9 | 8.9 | 8.9 | 8.9 |

**Table III (continued)**

| Example/comparison example no. | | 14 | 15 | E | 16 | 17 | F |
|---|---|---|---|---|---|---|---|
| Batch with | | | | | | | |
| Soya extracted meal, g | | 200 | 200 | 200 | | | |
| Milk yield feed, g | | | | | 200 | 200 | 200 |
| End product from ex. 5 (79.1% methionine), g | | 3.03 | | | 3.03 | | |
| End product from ex. 4 (86.8% methionine), g | | | 2.76 | | | 2.76 | |
| Product A¹ (86.6% methionine) | | | | 2.77 | | | 2.77 |
| Methionine content (calculated), % | | 1.2 | 1.2 | 1.2 | 1.2 | 1.2 | 1.2 |

¹Product A = protected methionine, pellet-shaped approx. 1.8 * 3.5 mm, analogously to ex. 11 from EP 0 495 349.

**TABLE IV**

| PARTICLE SIZE DISTRIBUTION AND BULK DENSITY OF THE FEEDSTUFFS EMPLOYED | | | | | |
|---|---|---|---|---|---|
| Particle size distribution | | Blattin M24 ADE | Spur-a-min | Extracted soya KFW | Milk yield feed ML 183 |
| < 100 $\mu$m | [%] | 4.4 | 20.8 | 3.2 | 8.4 |
| 100 - 150 $\mu$m | [%] | 3.6 | 7.1 | 1.2 | 4.6 |

(continued)

| PARTICLE SIZE DISTRIBUTION AND BULK DENSITY OF THE FEEDSTUFFS EMPLOYED | | | | | |
|---|---|---|---|---|---|
| Particle size distribution | | Blattin M24 ADE | Spur-a-min | Extracted soya KFW | Milk yield feed ML 183 |
| 150-200 $\mu$m | [%] | 5.0 | 8.0 | 1.3 | 5.5 |
| 200 -300 $\mu$m | [%] | 10.6 | 18.0 | 3.7 | 11.3 |
| 300 -500 $\mu$m | [%] | 15.5 | 156 | 8.9 | 17.2 |
| 500 -710 $\mu$m | [%] | 14.8 | 9.0 | 9.8 | 11.9 |
| 710 -1000 $\mu$m | [%] | 21.5 | 11.2 | 15.4 | 12.2 |
| 1000 -1400 $\mu$m | [%] | 18.5 | 8.2 | 22.6 | 14.6 |
| 1400 -2000 $\mu$m | [%] | 5.6 | 1.7 | 18.7 | 7.3 |
| 2000 -3150 $\mu$m | [%] | 0.6 | 0.4 | 12.2 | 7.0 |
| > 3150 $\mu$m | [%] | | | 30 | |
| | | | | | |
| Bulk density | [g/L] | 1009 | 833 | 618 | 552 |

TABLE V

| DEMIXING OF PROTECTED METHIONINE ACTIVE COMPOUND FORMULATIONS DURING TRICKLING | | | | | |
|---|---|---|---|---|---|
| Example / comparison example no. | Feedstuff | Protected methionine from example no. | Methionine content in the poured cone, [%] | | VC, [%] |
| 10 | Blattin M24 ADE | Ex. 5 | top : | 8.9 | 7 |
| | | (platelets) | middle : | 10.0 | |
| | | | bottom : | 10.0 | |
| 11 | | Ex. 4 | top : | 7.7 | 9 |
| | | (round particles) | middle : | 8.8 | |
| | | | bottom : | 9.1 | |
| C | Blattin M24 ADE | product A[1] | top : | 12.8 | 60 |
| | | | middle : | 2.9 | |
| | | | bottom : | 9.9 | |
| 12 | Spur-a-min | Ex. 5 | top : | 8.1 | 28 |
| | | (platelets) | middle : | 8.4 | |
| | | | bottom : | 13.1 | |
| 13 | | Ex. 4 | top : | 6.8 | 38 |
| | | (round particles) | middle : | 6.5 | |
| | | | bottom : | 12.3 | |
| D | Spur-a-min | product A[1] | top : | 6.2 | 70 |
| | | | middle : | 3.5 | |
| | | | bottom : | 14.3 | |
| 14 | Soya extracted meal KFW | Ex. 5 | top : | 1.3 | 46 |

(continued)

| DEMIXING OF PROTECTED METHIONINE ACTIVE COMPOUND FORMULATIONS DURING TRICKLING | | | | | |
|---|---|---|---|---|---|
| Example / comparison example no. | Feedstuff | Protected methionine from example no. | Methionine content in the poured cone, [%] | | VC, [%] |
| | | (platelets) | middle : | 1.5 | |
| | | | bottom : | 0.5 | |
| - 15 | | Ex. 4 | top : | 0.9 | 38 |
| | | (round particles) | middle : | 1.3 | |
| | | | bottom : | 0.6 | |
| E | | product A[1] | top : | 1.1 | 31 |
| | | | middle : | 0.9 | |
| | | | bottom : | 1.7 | |
| 16 | Milk yield feed ML 183 | Ex. 5 | top : | 1.2 | 18 |
| | | (platelets) | middle : | 1.3 | |
| | | | bottom : | 0.9 | |
| 17 | | Ex. 4 | top : | 0.8 | 7 |
| | | (round particles) | middle : | 0.8 | |
| | | | bottom : | 0.9 | |
| F | | product A[1] | top : | 0.9 | 27 |
| | | | middle : | 1.1 | |
| | | | bottom : | 1.5 | |
| [1]Product A = protected methionine, pellet-shaped approx. 1.8 * 3.5 mm, analogously to example 11 from EP 0 495 349 | | | | | |

The greater the differences in the contents analyzed within the poured cone, the greater the demixing has been during trickling. The variation coefficient of the content determination therefore serves as a measure of the demixing during trickling.

The comparison of examples 10 and 11 with comparison example C or examples 12 and 13 with comparison example D shows, in mixtures of the mineral feeds Blattin and Spur-a-min with the protected active compound formulations according to the invention, significantly lower variation coefficients of 7 - 9% and 28 - 38% respectively compared with conventional products with 60 and 70% respectively.

In the case of soya extraction meal as the carrier material, comparable VC values were still found with 38 - 46% in comparison with 31% (examples 14 and 15 compared with comparison example E).

In milk yield feed, the VC values of the active compound formulations according to the invention of 7-18% were in turn more favorable than in the case of conventional products with 27% (examples 16 and 17 compared with comparison example F).

The significantly lower demixing of the active compound formulations according to the invention during trickling increases the certainty that the animals fed are supplied more uniformly with the feed starting substances in question, and therefore contributes towards better feeding results, which is of decided advantage from economic and ecological aspects.

**Claims**

1. An active compound formulation protected by a coating, which comprises as the active compound an amino acid or salts thereof, is provided with at least one protective shell, has a particle diameter of a maximum of 1000 $\mu$m, and delayed release properties, wherein the protective shell consists of a single coating material selected from the group consisting of cellulose esters, ethers, acrylates, poly(meth)acrylates, polyamides, polyesters or copolymers of acrylonitrile, styrene, ethylene, propylene, butadiene, or esters and amides of methacrylic acid or acrylic acid.

2. An active compound formulation according to claim 1, wherein the protective shell is coated by one layer-application.

3. An active compound formulation according to claim 1 or 2, wherein the enclosed active compounds are present in the form of crystals or granules or spray granules.

4. An active compound formulation as claimed in claim 3, which is present in platelet-shaped particles or crystals, or round crystals.

5. An active compound formulation according to one or more of claim 1 to 4, wherein the active compound is methionine.

6. An active compound formulation according to one or more of claims 1 to 4, wherein active compounds are methionine salts which are suitable for feedstuffs use, chosen from the group Na, K, Ca, Mg, Zn methioninate in pure or mixed form.

7. An active compound formulation, according to one or more of claims 1 to 6, wherein the protective shell consists of cellulose ether.

8. An active compound formulation according to claim 7, wherein the protective shell consists of ethylcellulose.

9. An active compound formulation according to claim 8, wherein the amount of the protective shell is 4 - 30 wt.%, based on the end product.

10. An active compound formulation according to claim 9, wherein the amount of the protective shell is 5 - 20 wt.%, based on the end product.

11. An active compound formulation according to one or more of claims 1 to 10, wherein the particle diameters are in the range of between 100 and 1000 $\mu$m.

12. An active compound formulation according to claim 11, wherein the particle diameters are in the range of between 200 and 800 $\mu$m.

13. An active compound formulation according to claim 12, wherein the particle diameter is between 400 and 600 $\mu$m.

14. An active compound formulation according to one or more of claims 1 to 13 wherein the active compounds are methionine containing particles and the protective shell consists of ethylcellulose.

15. An active compound formulation according to claim 14, which has protection rates in the in vitro release test after 6 h of 60 - 90%, and after 6-24 h of 10 - 60% after 24 h.

16. An active compound formulation according to claim 15, which have protection rates in the in vitro release test after 6 h of 75 - 85%, and after 6-24 h of 35 - 50% after 24 h.

17. A process for the preparation of protected active compounds of amino acids, vitamins, pigments or enzymes by coating, which comprises providing the active compounds having a particle diameter of less than 1000 $\mu$m with a protective shell consisting of one coating material selected from the group consisting of cellulose esters, ethers, acrylates, poly(meth)acrylates, polyamides, polyesters or copolymers of acrylonitrile, styrene, ethylene, propylene, butadiene, or esters and amides of methacrylic acid or acrylic acid by fluidized bed coating or coacervation, and obtaining products with a particle diameter of a maximum of 1000 $\mu$m.

18. A process according to claim 17, wherein the protective shell is applied by one-layer application.

19. A process according to claim 17 or 18, wherein the active compounds are present in crystalline or granulated or spray granulated form.

20. A process according to one or more of claims 17 to 19 wherein the active compound is methionine or one or more salts of methionine.

21. A process as claimed in claim 20, wherein methionine is employed in the form of platelet-shaped particles or crystals, round crystals, granules or in the form of spray granules of methionine salts which are suitable for feedstuffs use, such as Na, K, Ca, Mg, Zn methioninate in pure or mixed form.

22. A process according to one or more of claims 17 to 21, wherein the protective shell consists of ethylcellulose.

23. A use of active compounds formulations as claimed in one or more of claims 1-16 for the nutrition of ruminants.

24. A use of active compounds formulations as claimed in one or more of claims 1-16 for the preparation of feedstuffs for ruminants.

**Patentansprüche**

1. Durch eine Beschichtung geschützte Wirkstoffzubereitung, die als Wirkstoff eine Aminosäure oder deren Salze enthält, mit mindestens einer Schutzhülle versehen ist, einen Partikeldurchmesser von maximal 1000 $\mu$m und ein verzögertes Freisetzungsverhalten aufweist, wobei die Schutzhülle aus einem einzigen Beschichtungsmaterial, ausgewählt aus der Gruppe, bestehend aus Celluloseestern, -ethern, Acrylaten, Poly(meth)acrylaten, Polyamiden, Polyestern bzw. Copolymeren aus Acrylnitril, Styrol, Ethylen, Propylen, Butadien oder Estern und Amiden der Methacrylsäure oder Acrylsäure, besteht.

2. Wirkstoffzubereitung nach Anspruch 1, bei der die Schutzhülle im Einschichtauftrag beschichtet ist.

3. Wirkstoffzubereitung nach Anspruch 1 oder 2, bei der die umhüllten Wirkstoffe in Form von Kristallen oder Granulaten oder Sprühgranulaten vorliegen.

4. Wirkstoffzubereitung nach Anspruch 3 in plättchenförmigen Partikeln oder Kristallen oder runden Kristallen.

5. Wirkstoffzubereitung nach einem oder mehreren der Ansprüche 1 bis 4, bei der es sich um Methionin handelt.

6. Wirkstoffzubereitung nach einem oder mehreren der Ansprüche 1 bis 4, bei der es sich um futtermitteltaugliche Methioninsalze, ausgewählt aus der Gruppe Na-, K-, Ca-, Mg-, Zn-Methioninat in reiner oder gemischter Form handelt.

7. Wirkstoffzubereitung nach einem oder mehreren der Ansprüche 1 bis 6, bei der die Schutzhülle aus Celluloseether besteht.

8. Wirkstoffzubereitung nach Anspruch 7, bei der die Schutzhülle aus Ethylcellulose besteht.

9. Wirkstoffzubereitung nach Anspruch 8, bei der die Menge der Schutzhülle 4-30 Gew.-%, bezogen auf das Endprodukt, beträgt.

10. Wirkstoffzubereitung nach Anspruch 9, bei der die Menge der Schutzhülle 5-20 Gew.-%, bezogen auf das Endprodukt, beträgt.

11. Wirkstoffzubereitung nach einem oder mehreren der Ansprüche 1 bis 10, bei der die Partikeldurchmesser im Bereich zwischen 100 und 1000 $\mu$m liegen.

12. Wirkstoffzubereitung nach Anspruch 11, bei der die Partikeldurchmesser im Bereich zwischen 200 und 800 $\mu$m liegen.

13. Wirkstoffzubereitung nach Anspruch 12, bei der die Partikeldurchmesser im Bereich zwischen 400 und 600 $\mu$m liegen.

14. Wirkstoffzubereitung nach einem oder mehreren der Ansprüche 1 bis 13, bei der es sich um Methionin enthaltende Partikel handelt und die Schutzhülle aus Ethylcellulose besteht.

**15.** Wirkstoffzubereitung nach Anspruch 14 mit Schutzraten im in vitro Freisetzungstest nach 6 h von 60-90% und nach 6-24 h von 10-60% nach 24 h.

**16.** Wirkstoffzubereitung nach Anspruch 15 mit Schutzraten im in vitro Freisetzungstest nach 6 h von 75-85% und nach 6-24 h von 35-50% nach 24 h.

**17.** Verfahren zur Herstellung von geschützten Wirkstoffzubereitungen von Aminosäuren, Vitaminen, Pigmenten oder Enzymen durch Beschichtung, bei dem man die Wirkstoffe mit einem Partikeldurchmesser von kleiner 1000 $\mu$m mit einer Schutzhülle, bestehend aus einem Beschichtungsmaterial, ausgewählt aus der Gruppe, bestehend aus Celluloseestern, -ethern, Acrylaten, Poly(meth)acrylaten, Polyamiden, Polyestern bzw. Copolymeren aus Acrylnitril, Styrol, Ethylen, Propylen, Butadien oder Estern und Amiden der Methacrylsäure oder Acrylsäure, durch Wirbelschichtbeschichtung oder Koazervation versieht und Produkte mit einem Partikeldurchmesser von maximal 1000 $\mu$m erhält.

**18.** Verfahren nach Anspruch 17, bei dem man die Schutzhülle im Einschichtauftrag aufträgt.

**19.** Verfahren nach Anspruch 17 oder 18, bei dem die Wirkstoffe in kristalliner oder granulierter oder sprühgranulierter Form vorliegen.

**20.** Verfahren nach einem oder mehreren der Ansprüche 17 bis 19, bei dem man als Wirkstoff Methionin oder eines oder mehrere der Salze von Methionin einsetzt.

**21.** Verfahren nach Anspruch 20, bei dem man Methionin in Form von plättchenförmigen Partikeln oder Kristallen, runden Kristallen, Granulaten oder in Form von Sprühgranulaten von futtermitteltauglichen Methioninsalzen wie Na-, K-, Ca-, Mg-, Zn-Methioninat in reiner oder gemischter Form einsetzt.

**22.** Verfahren nach einem oder mehreren der Ansprüche 17 bis 21, bei dem die Schutzhülle aus Ethylcellulose besteht.

**23.** Verwendung von Wirkstoffzubereitungen gemäß einem oder mehreren der Ansprüche 1 bis 16 zur Ernährung von Wiederkäuern.

**24.** Verwendung von Wirkstoffzubereitungen gemäß einem oder mehreren der Ansprüche 1 bis 16 zur Herstellung von Futtermitteln für Wiederkäuer.

**Revendications**

**1.** Formulation de composé actif protégée par un revêtement, qui comprend comme composé actif un aminoacide ou des sels de ce composé, est dotée d'au moins une enveloppe protectrice, possède un diamètre de particule de 1000 $\mu$m au maximum et des propriétés de libération retardée, dans laquelle l'enveloppe protectrice est constituée d'un seul matériau de revêtement choisi dans le groupe constitué par les esters de cellulose, les éthers, les acrylates, les poly(méth)acrylates, les polyamides, les polyesters ou les copolymères d'acrylonitrile, de styrène, d'éthylène, de propylène, de butadiène, ou d'esters et d'amides d'acide méthacrylique ou d'acide acrylique.

**2.** Formulation de composé actif selon la revendication 1, dans laquelle l'enveloppe protectrice est appliquée par application d'une seule couche.

**3.** Formulation de composé actif selon la revendication 1 ou 2, dans laquelle les composés actifs enfermés sont présents sous forme de cristaux ou de granulés ou de granulés d'atomisation.

**4.** Formulation de composé actif selon la revendication 3, qui est présente dans des particules ou cristaux lamellaires, ou des cristaux ronds.

**5.** Formulation de composé actif selon l'une au moins des revendications 1 à 4, dans laquelle le composé actif est la méthionine.

**6.** Formulation de composé actif selon l'une au moins des revendications 1 à 4, dans laquelle les composés actifs sont des sels de méthionine qui conviennent pour une utilisation alimentaire, choisis dans le groupe du méthioninate de Na, K, Ca, Mg, Zn sous forme pure ou sous forme de mélange.

7. Formulation de composé actif selon l'une au moins des revendications 1 à 6, dans laquelle l'enveloppe protectrice est constituée d'éther de cellulose.

8. Formulation de composé actif selon la revendication 7, dans laquelle l'enveloppe protectrice est constituée d'éthylcellulose.

9. Formulation de composé actif selon la revendication 8, dans laquelle la quantité d'enveloppe protectrice est de 4 - 30% en poids, par rapport au produit final.

10. Formulation de composé actif selon la revendication 9, dans laquelle la quantité d'enveloppe protectrice est de 5 - 20% en poids, par rapport au produit final.

11. Formulation de composé actif selon l'une au moins des revendications 1 à 10, dans laquelle les diamètres des particules sont dans la gamme comprise entre 100 et 1 000 $\mu$m.

12. Formulation de composé actif selon la revendication 11, dans laquelle les diamètres des particules sont dans la gamme comprise entre 200 et 800 $\mu$m.

13. Formulation de composé actif selon la revendication 12, dans laquelle le diamètre de particule est compris entre 400 et 600 $\mu$m.

14. Formulation de composé actif selon l'une au moins des revendications 1 à 13, dans laquelle les composés actifs sont des particules contenant de la méthionine et l'enveloppe protectrice est constituée d'éthylcellulose.

15. Formulation de composé actif selon la revendication 14, qui présente des taux de protection dans l'essai de libération in vitro après 6 h de 60 - 90%, et après 6 - 24 h de 10 - 60% après 24 h.

16. Formulation de composé actif selon la revendication 15, qui présente des taux de protection dans l'essai de libération in vitro après 6 h de 75 - 85%, et après 6 - 24 h de 35 - 50% après 24 h.

17. Procédé de préparation de composés actifs protégés d'aminoacides, de vitamines, de pigments ou d'enzymes par un revêtement, qui comprend le fait de doter les composés actifs ayant un diamètre de particule inférieur à 1 000 $\mu$m d'une enveloppe protectrice constituée d'un matériau de revêtement choisi dans le groupe constitué par les esters de cellulose, les éthers, les acrylates, les poly-(méth)acrylates, les polyamides, les polyesters ou les copolymères d'acrylonitrile, de styrène, d'éthylène, de propylène, de butadiène, ou d'esters et d'amides d'acide méthacrylique ou d'acide acrylique, par revêtement en lit fluidisé ou coacervation, et le fait d'obtenir des produits ayant un diamètre de particule de 1 000 $\mu$m au maximum.

18. Procédé selon la revendication 17, dans lequel l'enveloppe protectrice est appliquée par application d'une seule couche.

19. Procédé selon la revendication 17 ou 18, dans lequel les composés actifs sont présents sous forme cristalline ou sous forme de granulés ou de granulés atomisés.

20. Procédé selon l'une au moins des revendications 17 à 19, dans lequel le composé actif est la méthionine ou un ou plusieurs sels de méthionine.

21. Procédé selon la revendication 20, dans lequel la méthionine est employée sous forme de particules ou de cristaux lamellaires, de cristaux ronds, de granulés ou sous forme de granulés d'atomisation de sels de méthionine qui conviennent pour une utilisation alimentaire, tels que le méthioninate de Na, K, Ca, Mg, Zn, sous forme pure ou sous forme de mélange.

22. Procédé selon l'une au moins des revendications 17 à 21, dans lequel l'enveloppe protectrice est constituée d'éthylcellulose.

23. Utilisation de formulations de composés actifs selon l'une au moins des revendications 1 à 16 pour la nutrition de ruminants.

24. Utilisation de formulations de composés actifs selon l'une au moins des revendications 1 à 16 pour la préparation d'aliments pour ruminants.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0495349 A
- US 4877621 A
- US 4876097 A
- EP 427639 A

- EP 406041 A
- EP 447297 A
- EP 0614615 A
- US 5290560 A
- EP 588346 A
- EP 495349 A